(19) Europäisches Patentamt
European Patent Office
Office européen des brevets

(11) **EP 4 202 054 A1**

(12) **EUROPEAN PATENT APPLICATION**
published in accordance with Art. 153(4) EPC

(43) Date of publication:
**28.06.2023  Bulletin 2023/26**

(21) Application number: **21861575.5**

(22) Date of filing: **24.08.2021**

(51) International Patent Classification (IPC):
*C12P 21/00* (2006.01)    *C12N 9/50* (2006.01)
*C12N 9/78* (2006.01)    *C12N 9/90* (2006.01)
*C12P 21/06* (2006.01)

(52) Cooperative Patent Classification (CPC):
**C12N 9/50; C12N 9/78; C12N 9/90; C12P 21/00; C12P 21/06**

(86) International application number:
**PCT/JP2021/031039**

(87) International publication number:
**WO 2022/045151 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.08.2020  JP 2020141053**

(71) Applicant: **Amano Enzyme Inc.**
**Nagoya-shi**
**Aichi 460-8630 (JP)**

(72) Inventors:
• **MATSUBARA, Hirotaka**
  **Kakamigahara-shi, Gifu 509-0109 (JP)**
• **SAKAI, Kiyota**
  **Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

Remarks:
The complete document including Reference Table(s) and the Sequence Listing(s) can be downloaded from the EPO website

(54)    **METHOD FOR PRODUCING MODIFIED PROTEIN**

(57)    The present invention addresses the problem of providing a new means capable of modifying a protein material. According to the present invention, a protein material can be modified in property by causing a peptidylprolyl isomerase, or a peptidylprolyl isomerase and a protein modification enzyme to act on the protein material.

EP 4 202 054 A1

**Description**

TECHNICAL FIELD

The present invention relates to a method for producing a modified protein and a method for producing a modified protein material.

BACKGROUND ART

A peptidylprolyl isomerase (hereinafter, it may be abbreviated as "PPIase") acts on a peptide bond on the N-terminal side of a proline (Pro) residue in a peptide or protein, and catalyzes cis-trans isomerization. PPIase has a so-called chaperone function involved in protein folding (see, for example, Non-Patent Document 1). The chaperone function of PPIase is utilized, for example, for improving expression efficiency of a heterologous protein in a host cell (see, for example, Patent Documents 1 to 3). On the other hand, PPIase is also used for screening for immunosuppressive substances (see, for example, Patent Document 4). In addition, it is known that thermal stability of an enzyme can be improved by PPIase (see, for example, Patent Document 5). Non-Patent Document 2 discloses a PPIase activity measurement method utilizing the fact that chymotrypsin acts only on a trans form of a synthetic substrate (peptide).

On the other hand, researches for modifying protein materials by enzymes have been actively conducted. For modification of a protein material, molecular conversion reactions of protein molecules, such as protein degradation by protease or the like, protein side-chain modification by protein glutaminase or the like, intermolecular protein molecule crosslinking by transglutaminase or oxidase, are used, and such molecular conversion reactions enable characteristic changes (that is, modification) such as taste, physical properties, and functionality (for example, foaming property, emulsion stability, solubility, and the like) of the protein material. As a more specific aspect of such modification of a protein material, for example, techniques such as softening of meat by protease, binding of meat by transglutaminase, improvement of solubility of a protein material by protein glutaminase, and the like, are known, and a protein material modified by such a technique is provided as a new protein material.

PRIOR ART DOCUMENTS

PATENT DOCUMENTS

Patent Document 1: Unexamined Patent Application Publication (Translation of PCT Application) No. 2002-525120
Patent Document 2: Japanese Patent Laid-open Publication No. 2004-215591
Patent Document 3: Japanese Patent Laid-open Publication No. 2005-046066
Patent Document 4: Japanese Patent Laid-open Publication No. H2-124100
Patent Document 5: Japanese Patent Laid-open Publication No. H11-075837

NON-PATENT DOCUMENTS

Non-Patent Document 1: Biochim Biophys Acta. 2015 October; 1850(10): 2017-2034.

Non-Patent Document 2: Biomed Biochim Acta 1984;43(10):1101-11.

SUMMARY OF THE INVENTION

PROBLEMS TO BE SOLVED BY THE INVENTION

Since the modified protein material has improved properties or has a new function, it can be expected to be applied to various fields, and its utility value is high. There is still a high need for further means for providing such a high-utility protein material. Therefore, an object of the present invention is to provide a new means for modifying (processing) a protein material.

MEANS FOR SOLVING THE PROBLEM

The present inventor has found that PPIase itself enables modification of protein materials. In view of the fact that PPIase is only known to catalyze the reaction of isomerization, it was unexpected that the modification of a protein material, which has been performed by an enzyme that catalyzes the molecular conversion reaction of a protein molecule, is enabled by PPIase. Furthermore, it has also been found that reaction efficiency by a protein modification enzyme can be adjusted by using PPIase in combination with an enzyme (hereinafter, also described as "protein modification enzyme") that catalyzes a molecular conversion reaction of a protein molecule. The following invention is mainly based on the above findings.

[1] A method for producing a modified protein, comprising a step of allowing a peptidylprolyl isomerase and a protein modification enzyme to act on a protein.
[2] The production method according to [1], wherein the treatment of allowing the protein modification enzyme to act and the treatment of allowing the peptidylprolyl isomerase to act are performed simultaneously.
[3] The production method according to [1] or [2], wherein the protein modification enzyme is selected from the group consisting of a proteolytic enzyme, a protein side-chain modifying enzyme, and a protein crosslinking enzyme.
[4] The production method according to [1] or [2], wherein the protein modification enzyme is selected from the group consisting of a protease, a protein deamidase, a transglutaminase, and an oxidoreductase that catalyzes crosslinking of proteins.
[5] The production method according to any one of [1] to [4], wherein the protein is selected from the group consisting of plant proteins derived from beans, grains, nuts and seeds; milk proteins, egg proteins, blood proteins, muscle proteins and tendon proteins; and proteins derived from microorganisms, algae, and insects.
[6] A method for producing a property modified protein material, comprising a step of treating a food material, a pharmaceutical material, or an industrial material containing a protein with a peptidylprolyl isomerase and a protein modification enzyme.
[7] The production method according to [6], wherein the treatment with the protein modification enzyme is performed simultaneously with the treatment with the peptidylprolyl isomerase.
[8] An enzyme agent for protein modification, containing a peptidylprolyl isomerase and a protein modification enzyme.
[9] The enzyme agent for protein modification according to [8], wherein the protein modification enzyme is selected from the group consisting of a proteolytic enzyme, a protein side-chain modifying enzyme, and a protein crosslinking enzyme.
[10] The enzyme agent for protein modification according to [8], wherein the protein modification enzyme is selected from the group consisting of a protease, a peptidase, a protein deamidase, a transglutaminase, and an oxidoreductase that catalyzes crosslinking of proteins.
[11] A method for producing a property modified protein material, comprising a step of treating a food material, a pharmaceutical material or industrial material containing a protein with a peptidylprolyl isomerase.
[12] An enzyme agent for property modifying a protein material, containing a peptidylprolyl isomerase.

ADVANTAGES OF THE INVENTION

According to the present invention, a new means capable of modifying a protein material is provided.

BRIEF DESCRIPTION OF THE DRAWINGS

Fig. 1: Upper part: Evaluation of deamidation of soybean protein. Deamidation was compared between a case where PPIase (Cyclophilin A) was added (white bar) and a case where PPIase was not added (filled bar) when protein glutaminase was reacted. Lower part: Evaluation of deamidation of wheat gluten. Deamidation was compared between a case where PPIase (Cyclophilin A) was added (white bar) and a case where PPIase was not added (filled bar) when protein glutaminase was reacted.
Fig. 2 shows a residual ratio of a 33 mer peptide derived from wheat gluten after decomposition. The residual ratio (%) of the 33 mer peptide was compared between the case of reaction with pepsin alone (filled bar. corresponding to digestion of artificial gastric fluid) and the case of reaction with pepsin in combination with PPIase (Cyclophilin A) (outlined bar) (relative value with the residual ratio in the case of reacting pepsin alone as 100%).
Fig. 3 shows the properties of PPIase (SgPPI) derived from Streptomyces griseus.
Fig. 4 shows an effect when wheat gluten, soybean protein, pea protein, milk casein, or egg albumin is treated in combination with SgPPI and protein glutaminase.

Fig. 5 shows the results of confirming the presence or absence of an influence on protein glutaminase (PG) when SgPPI is used in combination with PG.

Fig. 6 shows an effect when milk casein is treated with SgPPI and laccase in combination.

Fig. 7 shows the effect of treating chickpea protein with PPIase alone.

EMBODIMENTS OF THE INVENTION

In the present specification, "modification" of a protein refers to molecular conversion by decomposition, synthesis, crosslinking, or the like, in other words, processing of changing the structure of a protein so as to change the number and/or type of constituent atoms of a protein molecule (also described as "protein substrate") (therefore, mere isomerization is not included in modification), and means structural-modification with respect to "property modification" described later. In addition, processing a protein-containing material (also referred to as "protein material") so that the properties of the protein material are changed based on a change in physical properties of the protein by subjecting the protein-containing material to enzyme treatment is referred to as" property modification". In the present invention, " property modification" of a protein material includes an action and effect based on "modification" of a protein, an action and effect based on isomerization of a protein by a peptidylprolyl isomerase, and an action and effect in which both the actions and effects are exerted in combination.

Furthermore, the "protein" in the present invention broadly includes a substance in which a plurality of amino acids are peptide-linked. Therefore, the "protein" in the present invention includes any of an oligopeptide having 10 or less amino acid residues, a polypeptide having more than 10 amino acid residues, and a protein in which the polypeptide forms a three-dimensional structure.

1. Method for producing modified protein

A first aspect of the present invention relates to a method for producing a modified protein. The method for producing a modified protein of the present invention is characterized by including a step of allowing a peptidylprolyl isomerase (PPIase) and a protein modification enzyme to act on a protein (hereinafter also referred to as "substrate protein").

Treatment of proteins with both PPIase and protein modification enzymes allows for regulation of the protein modification reaction by the protein modification enzymes. The regulation of protein treatment is upregulation (acceleration of reaction) in typical and preferred examples, which promotes the reaction of the protein modification enzyme. On the other hand, the regulation is also allowed to be downregulation (suppression of reaction), and in this case, the reaction between the PPIase and the protein modification enzyme is suppressed. A mode of such a downregulation is applied, for example, when there is a problem that the reaction proceeds too much (for example, when the protein modification enzyme is a crosslinking enzyme, the mouthfeel and the like are deteriorated due to excessive crosslinking, and when the protein modification enzyme is a decomposing (cleaving) enzyme, the taste and physical properties are deteriorated).

1-1. Protein (substrate protein)

There is no particular restriction on the origin of the protein (substrate protein) used in the present invention. Examples of the plant protein which is an example of the protein used in the present invention include proteins derived from seeds such as beans such as soy beans, green peas, lentils, chickpeas, black beans, and the like; cereals such as wheat, barley, rye, oat, rice, and the like; nuts such as almond, peanut, and the like; hemp seeds, chia seeds, Quinoa, Amaranthus, and the like. Examples of the animal protein which is another example of the protein used in the present invention include milk proteins such as casein, β-lactoglobulin, and the like; egg proteins such as ovalbumin and the like; blood proteins such as serum albumin and the like; muscle proteins such as myosin, actin, and the like; and tendon proteins such as gelatin, collagen, and the like. Other proteins which are further examples of proteins used in the present invention include proteins derived from insects such as cricket, silkworm, and the like; microorganisms such as yeast, filamentous fungi, and the like, and algae such as spirulina and the like.

Still another example of the protein used in the present invention includes pre-modified proteins such as a protein obtained by chemically partially decomposing the natural protein with an acid, an alkali, or the like, a protein obtained by enzymatically partially decomposing the natural protein with a protease or the like, a protein obtained by chemically modifying the natural protein with various reagents and the like, in addition to the natural proteins described above.

The protein to be treated in the present invention includes not only proteins that are industrially useful (that is, the treatment according to the present invention provides new characteristics, thereby enhancing the usefulness) but also proteins that are not industrially useful (that is, by reducing or eliminating undesired properties possessed by the protein by the treatment according to the present invention, usefulness is newly imparted). Examples of proteins that are not industrially useful include various allergens (gluten and the like contained in grain such as wheat, barley, rye, oat and the like, etc.), toxic peptides (examples thereof include a 33 mer gliadin peptide and the like contained in a gluten decomposition product), exogenous opioid peptides (Examples thereof include casomorphin contained in a casein decomposition product, gliadorphin contained in a gluten decomposition product, etc.), and the like.

1-2. Peptidvlprolvl Isomerase (PPIase)

PPIase is an enzyme that acts on a peptide bond on the N-terminal side of a proline (Pro) residue in a peptide or protein and catalyzes cis-trans isomerization. In other words, PPIase has an activity of changing the peptide bond on the N-terminal side of the Pro residue in the peptide or protein to a cis/trans form (PPIase activity). The PPIase used in the present invention is not particularly limited as long as it is a polypeptide having the activity. Therefore, even if the polypeptide is not classified as PPIase in the official database, the polypeptide having PPIase activity corresponds to PPIase used in the present invention.

The method for detecting the presence or absence of PPIase activity includes a method in which PPIase and chymotrypsin are added to a synthetic substrate Suc-Ala-Ala-Pro-Phe-pNA (Suc-AAPF-pNA), and pNA release is detected; a method for detecting fluorescence of Abz by adding Abz-Ala-Xaa-Pro-Phe-pNA, PPIase and protease; an activity detection method using a FRET (Fluorescence Resonance Energy Transfer) substrate; and the like, and when the PPIase activity can be detected by at least any one of these methods, it can be determined that the enzyme has the PPIase activity. Preferably, the presence or absence of PPIase activity can be confirmed by a method for detecting pNA release among the above methods. In the present invention, a specific value of activity of PPIase is measured based on a method for detecting release of pNA.

The origin of PPIase used in the present invention is not particularly limited, and examples thereof include a wide range of organisms including prokaryotes, eukaryotes, and archaea (Biochim Biophys Acta. 2015 Oct; 1850 (10): 2017 -2034). The activity of PPIase can be provided as long as there is a characteristic pocket (cavity) having a diameter of 10 Å and a depth of 6 Å (Protein Engineering, Design and Selection, Volume 21, Issue 2, February 2008, pp 83 -89) regardless of the presence of the side chain, and thus a polypeptide having the pocket can be used as the PPIase of the present invention. Specific examples of the polypeptide having PPIase activity include cyclophilin (Cyclophilin A or the like), FKBP (FKBP 12 or the like), Pin1, parvulin, polypeptides of the following [1] to [3], and the like.

[1] A polypeptide containing the amino acid sequence set forth in SEQ ID NO: 1
[2] A polypeptide containing an amino acid sequence in which one or several amino acids are substituted, added, inserted, or deleted in the amino acid sequence set forth in SEQ ID NO: 1, and having a PPIase activity
[3] A polypeptide containing an amino acid sequence having 68% or more of sequence identity to the amino acid sequence set forth in SEQ ID NO: 1, and having PPIase activity

SEQ ID NO: 1 in [1] is an amino acid sequence of PPIase from Streptomyces griseus.
The polypeptides of [2] and [3] are sequence-similar PPIases having the amino acid sequence of the polypeptide of [1] as a basic skeleton.
The modification of the amino acid to be introduced into the polypeptide of [2] may include only one modification (for example, substitution) from among substitution, addition, insertion, and deletion, or may include two or more modifications (for example, substitution and insertion). In the polypeptide of [2], the number of amino acids to be substituted, added, inserted or deleted may be 1 or more or several, and is, for example, 1 to 97, 1 to 80, 1 to 60, 1 to 40, 1 to 20, 1 to 10, preferably 1 to 8, 1 to 6, 1 to 5, or 1 to 4, more preferably 1 to 3, and particularly preferably 1 or 2, or 1.
In the polypeptide of [3], the sequence identity may be 68% or more, but is preferably 80% or more, more preferably 90% or more, still more preferably 95% or more, even more preferably 98% or more, further even more preferably 99% or more, particularly preferably 99.5% or more, and most preferably 99.8% or more.
Here, in the polypeptide of [3], for example, the sequence identity to the amino acid sequence shown in SEQ ID NO: 1 is a sequence identity calculated as compared with the amino acid sequence shown in SEQ ID NO: 1. In addition, the "sequence identity" indicates a value of identity of an amino acid sequence obtained by bl2seq program (Tatiana A. Tatsusova, Thomas L. Madden, FEMS Microbiol. Lett., Vol. 174, p 247 -250, 1999) of BLAST PACKAGE [sgi 32 bit edition, Version 2.0.12; available from National Center for Biotechnology Information (NCBI)]. The parameters may be set to Gap insertion Cost value: 11 and Gap extension Cost value: 1.
In the polypeptides of [2] and [3], positions 41 to 165 and positions 220 to 309 in the amino acid sequence shown in SEQ ID NO: 1 constitute an FK 506 binding protein (FKBP) domain, and therefore it is desirable not to introduce substitutions or deletions at these sites.
When an amino acid substitution is introduced into the polypeptides of [2] and [3], a conservative substitution can be mentioned as a mode of the amino acid substitution. That is, in the polypeptides of [2] and [3], examples of the amino acid substitution introduced into the amino acid sequence shown in SEQ ID NO: 1 include a substitution with another non-polar amino acid if the amino acid before substitution is a non-polar amino acid, a substitution with another non-charged amino acid if the amino acid before substitution is a non-charged amino acid, a substitution with another acidic amino acid if the amino acid before substitution is an acidic amino acid, and a substitution with another basic amino acid if the amino acid before substitution is a basic amino acid.

When an amino acid addition is introduced into the polypeptides of [2] and [3], examples of a mode of the amino acid addition include addition of a methionine residue to the N-terminal, addition of a tag for purification (for example, a binding oligopeptide such as oligohistidine or the like), and the like.

The polypeptides of [2] and [3] include not only polypeptides obtained by artificial mutation but also polypeptides generated by naturally occurring mutations (mutants or variants) based on individual differences or species differences in an organism from which the polypeptides are derived.

Furthermore, the method for confirming the "PPIase activity" in the polypeptides of [2] and [3] is as described above. Preferably, the "PPIase activity" in the polypeptides of [2] and [3] is equivalent to the polypeptide of [1], and specifically, the amount of free pNA measured for the polypeptides of [2] and [3] based on the method for detecting release of pNA from the synthetic substrate Suc-Ala-Ala-Pro-Phe-pNA (Suc-AAPF-pNA) in the presence of chymotrypsin is 80 to 120%, and preferably 90 to 110%, of the amount of free pNA measured for the polypeptide of [1].

Some PPIases are commercially available (for example, FKBP 12 from Sigma and Cyclophilin A from Sigma) and are readily available. These PPIases may be used singly or in combination of two or more kinds thereof.

The amount of PPIase used is, for example, 0.0001 mg to 1 g, preferably 0.01 mg to 100 mg, or, for example, 0.01 to 100000 U, preferably 0.1 to 10000 U, more preferably 1 to 1000 U, further preferably 10 to 200 U, as an amount per 1 g of the substrate protein.

## 1-3. Protein modification enzyme

In the method for producing a modified protein of the present invention, a protein modification enzyme (protein processing enzyme) is used in combination with PPIase. That is, PPIase is allowed to act on a substrate protein, and a protein modification enzyme is also allowed to act.

The protein modification enzyme is an enzyme capable of modifying (Processing of changing the structure of the protein so as to change the number and/or type of constituent atoms of the protein molecule can be performed) a substrate protein molecule by its catalytic action. Examples of the protein modification enzyme include proteolytic enzymes, protein side-chain modifying enzymes, and protein crosslinking enzymes. Specific examples of the proteolytic enzyme include proteases (Pepsin, trypsin, chymotrypsin, papain, metal protease, serine protease, cysteine protease, proteinase such as acid protease or the like, carboxypeptidase, aminopeptidase, etc.). Specific examples of the protein side-chain modifying enzyme include protein deamidases (protein glutaminase, protein arginine deiminase, and the like, and transglutaminase). Specific examples of the protein crosslinking enzyme include oxidoreductases (lysyl oxidase, sulfhydryl oxidase, tyrosinase, laccase, bilirubin oxidase, ascorbic acid oxidase, ceruloplasmin, peroxidase, and the like) that catalyze crosslinking of proteins.

The protein deamidase is an enzyme that deamides an amide group of a glutamine residue and an asparagine residue in a protein. As an enzyme that deamides a glutamine residue in a protein, for example, protein glutaminase derived from Chryseobacterium proteolyticum (Eur J Biochem, 268 (5), 1410, 2001, Protein-glutaminase From Chryseobacterium Proteolyticum, an Enzyme That Deamidates Glutaminyl Residues in Proteins.Purification, Characterization and Gene Cloning, S Yamaguchi 1, D J Jeenes, D B Archer, or Front Microbiol, 9, 1975, 2018, Complete Genome Sequence and Characterization of a Protein-Glutaminase Producing Strain, Chryseobacterium proteolyticum QSH 1265, Ruidan Qu, Xiaoyu Zhu, Min Tian, Yingjie Liu, Wenjuan Yan, Jian Ye, Hongliang Gao, Jing Huang Yang) is well known, but the enzyme is not limited thereto. An enzyme that deamides an asparagine residue in a protein is disclosed in, for example, WO 2015/133590, but is not limited thereto. The protein deamidase referred to in the present invention also includes an enzyme that deiminates an arginine residue. As an enzyme that deiminates an arginine residue, for example, arginine deiminase derived from Fusarium graminearum is known.

In general, when glutamine and asparagine residues in a protein are deamidated to generate a carboxyl group, the negative charge of the protein increases, and as a result, the isoelectric point decreases and the hydration force increases. Furthermore, an increase in electrostatic repulsive force leads to a decrease in interaction between proteins, that is, a decrease in associative properties. These changes greatly increase the solubility and water dispersibility of the protein. In addition, the increase in the negative charge of the protein loosens the folding of the protein, changes the higher-order structure, and exposes the hydrophobic region buried inside the molecule to the surface of the molecule. Therefore, the deamidated protein has amphiphilicity and becomes an ideal surfactant, and the emulsifying power, emulsion stability, foaming property, and foam stability of the protein are greatly improved. Thus, deamidation of a protein leads to an improvement in various functional properties of the protein, and the use of the protein is dramatically increased (for example, Molecular Approaches to Improving Food Quality and Safety, D. Chatnagar and T. E. Cleveland, eds., Van Nostrand Reinhold, New York, 1992, p. 37). Also, when an arginine residue in a protein is deiminated, the hydrophobicity of the protein is increased to change the higher order structure of the protein.

The transglutaminase is an enzyme that catalyzes an acyl transfer reaction of a γ-carboxylamide group of a glutamine residue in a peptide chain, and when an ε-amino group of a lysine residue in a protein acts as an acyl receptor, an ε-(γ-Gln)-Lys bridge bond is formed in or between molecules of a protein molecule. Since protein property modification can be performed by utilizing the action of transglutaminase, transglutaminase derived from the genus Streptomyces is used for the meat biding, and production of sausages, bean curd, bread, and noodles. In addition, transglutaminase is used not only in the food field but also in the fiber field, the medical field, the cosmetic field, and the like.

The above-described protein modification enzymes may be used alone, or may be used in combination of two or more thereof. Examples of the combination of two or more kinds of protein modification enzymes include a combination of protein glutaminase and transglutaminase, a combination of protein glutaminase and laccase, a combination of transglutaminase and laccase, a combination of protease and transglutaminase, and the like.

The amount of the protein modification enzyme to be used per 1 g of the substrate protein is, for example, 0.001 mg to 1 g, preferably 0.01 mg to 0.1 g, or, for example, 0.001 to 10000 U, preferably 0.01 to 5000 U, and more preferably 0.1 to 3000 U.

1-4. Reaction operation, reaction conditions, etc.

The treatment of allowing the PPIase to act and the treatment of allowing the protein modification enzyme to act may be performed simultaneously or sequentially. In the present invention, from the viewpoint of work efficiency and/or reaction efficiency, the treatment with PPIase and the treatment with a protein modification enzyme are preferably performed simultaneously.

When the treatment with PPIase and the treatment with the protein modification enzyme are simultaneously performed, a state in which PPIase and the protein modification enzyme coexist in the substrate protein solution may be formed, and the start timing of the treatment may be the same or the start timing of either one of the treatments may be delayed. In addition, in a case where both processes are performed at the same time, the end timing of the processes may be the same time, or the end timing of one of the processes may be delayed. For example, when two or more protein modification enzymes are used in combination, all the protein modification enzymes may be used for the simultaneous reaction with PPIase, or some of the protein modification enzymes may be used for the simultaneous reaction with PPIase, and the remaining protein modification enzymes may be used after the simultaneous reaction.

The temperature condition and the pH condition in the treatment of the protein can be appropriately determined by those skilled in the art according to the optimum temperature and the optimum pH of the enzyme to be used. For both treatment with PPIase and treatment with a protein modification enzyme, the temperature condition is, for example, 10°C to 60°C, preferably 20°C to 50°C, and the pH condition is, for example, 3 to 9, preferably 4 to 8.

Regarding the reaction time required for the treatment of the protein, the total time required for the treatment with PPIase and the treatment with the protein modification enzyme is, for example, 1 minute to 24 hours, preferably 10 minutes to 16 hours.

Specifically, the optimum reaction conditions may be determined through a preliminary experiment.

1-5. Modified protein

The modified protein obtained by the method for producing a modified protein of the present invention is produced when the protein described in the above "1-1. Protein (substrate protein)" undergoes a modification action by the protein modification enzyme described in the above "1-3. Protein-modification enzyme". Specific examples of the modified protein include proteolytic products when a proteolytic enzyme is used as the protein modification enzyme, proteins subjected to side-chain modification when a protein side-chain modifying enzyme is used as the protein modification enzyme, and proteins crosslinked between molecules and/or within molecules when a protein crosslinking enzyme is used as the protein modification enzyme.

2. Method for producing property modified protein material

A second aspect of the present invention relates to a method for producing a property modified protein material. As described in the method for producing a modified protein according to the first aspect, since a combination of a peptidylprolyl isomerase and a protein modification enzyme can be used for modifying a substrate protein, it is possible to modify in property of a protein material by applying the combination to the treatment of the protein material. Furthermore, even when a peptidylprolyl isomerase is used alone, it is possible to modify in property of a protein material by applying the peptidylprolyl isomerase to the treatment of the protein material. Therefore, the present invention also provides a method for producing a property modified protein material.

Specifically, the method for producing a property modified protein material of the present invention includes a step of treating a food material, a pharmaceutical material, or an industrial material containing a protein with a peptidylprolyl isomerase or a peptidylprolyl isomerase and a protein modification enzyme.

The specific form of the property modification may be any form as long as it can be expected from the action and effect of the protein modification enzyme.

Examples of specific forms of the property modification include improvement of the water dispersibility of the protein in the protein material, improvement of the protein solubility in the protein material, improvement of the emulsion stability of the protein material, improvement of the internal digestibility of the protein material, improvement of the taste of the protein material, improvement of the foaming property of the protein material, improvement of the gelation property of the protein material, and the like, depending on the type and/or form of the protein material.

Other examples of specific forms of property modification also include reduction or elimination of allergenicity or opioidicity, imparting limited allergic properties useful for desensitization therapy (that is, reducing allergy and allowing it to remain within a predetermined limited range), and the like.

The protein material to be treated in the method for producing a property modified protein material of the present invention is a raw material that contains protein and is used for producing articles (specific examples include foods, pharmaceuticals, and industrial articles) distributed in various industries. The proteins contained in the protein material are as described in "1-1. Protein (substrate protein)" above. Specific examples of the protein material include food materials, pharmaceutical materials, and industrial materials. More specific examples of the food material include all food materials used as so-called food materials, such as animal tissues and plant tissues, and processed products such as dispersions (particularly preferred is plant milk in which a pulverized product of an edible part of a plant is dispersed in water) and extracts thereof, and, a composition containing an allergen and a sitologically acceptable ingredient, a composition containing an exogenous opioid peptide and a sitologically acceptable ingredient, a composition containing a toxic peptide and a sitologically acceptable ingredient, and the like. More specific examples of the pharmaceutical material include a composition containing an allergen and a pharmaceutically acceptable component, and the like. More specific examples of the industrial material include fibers such as silk, wool, and the like.

In the property modified protein material obtained by the method of producing a property modified protein material of the present invention is the result of the protein material undergoing the action of peptidyl prolyl isomerase or the action of peptidyl prolyl isomerase and a protein modifying enzyme.

Among the property modified protein material, examples of those resulting from the action of peptidyl prolyl isomerase on the protein material include protein materials (specifically, food materials or pharmaceutical materials) having improved protein dispersibility in water, improved protein solubility and/or improved emulsion stability, and the like.

Among the property modified protein material, examples of those resulting from the action of peptidyl prolyl isomerase and a protein modification enzyme on the protein material include protein materials having improved protein dispersibility in water, improved protein solubility, improved emulsion stability, improved foaming property, and/or improved gelation characteristics (specifically, food materials or pharmaceutical materials), protein materials having improved internal digestibility and/or improved taste (specifically, a food material), and protein materials in which allergic, opioid, toxicity, and/or unnecessary substances are reduced or eliminated (specifically, food materials, pharmaceutical materials, or industrial materials), and the like.

Among the above-described property modified protein materials, the food material having improved internal digestibility can be used for, for example, foods and beverages for animals having a reduced digestive function, health foods and beverages having enhanced digestive absorption ability, and the like; the food material in which the allergy is reduced or eliminated can be used for, for example, food and drink for animals having a diathesis causing an allergy to the allergen because the allergen is reduced or eliminated; the food material in which the opioid property is reduced or eliminated can be used for, for example, foods and beverages for animals suffering from a mental disease such as autism or the like caused by the opioid peptide, since the exogenous opioid peptide is reduced or eliminated; the food material having reduced or eliminated toxicity can be used for, for example, food and drink for animals suffering from coeliac disease and other gluten-related diseases, because the toxic peptide is reduced or eliminated; and the like.

Among the above-described property modified protein materials, the pharmaceutical material in which the allergy is reduced is adjusted such that a part of the allergen is decomposed or the like from the composition containing the allergen and the pharmaceutically acceptable component, and the allergen content remains within a predetermined limited concentration range, and thus, for example, the pharmaceutical material can be used as a medicine for desensitization therapy of allergy.

Among the above-described property modified protein materials, an industrial material in which allergy is reduced or eliminated can be used as a material of a textile product that touches the skin; and examples of the industrial material from which unnecessary substances have been reduced or eliminated include silk from which sericin has been removed (scoured), and wool from which scale has been removed, and the industrial material can be used as a material for a textile product with improved texture.

The type of the animal is not particularly limited, and examples thereof include mammals, birds, and the like, preferably mammals, and more preferably humans.

Details of the PPIase, the protein modification enzyme., the use amount of these enzymes, the reaction operation, the reaction conditions, and the like, which are applied in the method for producing a property modified protein material of the present invention, are as described in the method for producing a modified protein according to the first aspect.

3. Enzyme agent for protein modification

A third aspect of the present invention relates to an enzyme agent for protein modification. As described in the method for producing a modified protein according to the first aspect, a combination of a peptidylprolyl isomerase and a protein modification enzyme can be used for modifying a substrate protein. Therefore, the present invention also provides an enzyme agent for protein modification containing a peptidylprolyl isomerase and a protein modification enzyme.

In the enzyme agent for protein modification, the contents of PPIase and the protein modification enzyme as active ingredients are not particularly limited. For example, the content of PPIase per 1 g of the enzyme agent for protein modification is, for example, 0.0001 mg to 1000 mg, preferably 0.01 mg to 100 mg. When protease is used as the enzyme for protein modification, the content of the enzyme for protein modification per 1 g of the enzyme agent for protein modification is, for example, 0.0001 mg to 1000 mg, preferably 0.01 mg to 100 mg; when peptidase is used as the enzyme for protein modification, the content is, for example, 0.0001 mg to 1000 mg, preferably 0.01 mg to 100 mg; when protein glutaminase is used as the enzyme for protein modification, the content is, for example, 0.0001 mg to 1000 mg, preferably 0.01 mg to 100 mg per 1 g of the present enzyme agent; and when transglutaminase is used as the enzyme for protein modification, the content is, for example, 0.0001 mg to 1000 mg, preferably 0.01 mg to 100 mg.

The enzyme agent for protein modification of the present invention may contain other components such as an excipient, a buffer, a suspending agent, a stabilizer, a preservative, an antiseptic agent, physiological saline, and the like in addition to the enzymes as an active ingredient. Examples of the excipient include lactose, sorbitol, D-mannitol, maltodextrin, sucrose, and the like. Examples of the buffer include phosphates, citrates, acetates, and the like. Examples of the stabilizer include propylene glycol, ascorbic acid, and the like. Examples of the preservative include phenol, benzalkonium chloride, benzyl alcohol, chlorobutanol, methylparaben, and the like. Examples of the antiseptic agent include benzalkonium chloride, paraoxybenzoic acid, chlorobutanol, and the like.

The property of the enzyme agent for protein modification according to the present invention is not particularly limited, and examples thereof include a solid form, a liquid form, and a supported form. When the enzyme agent for protein modification of the present invention is in a solid form, more specific forms thereof include granules, powders, and the like. When the enzyme agent for protein modification of the present invention is in a supported state, a more specific form may be a form in which the enzyme as an active ingredient is immobilized on the surface of an insoluble carrier such as silica, porous polymer, and the like.

Details of the PPIase, the protein modification enzyme, the modification mode, the protein of interest, and the method of use used in the enzyme agent for protein modification of the present invention are as described in the method for producing a modified protein according to the first aspect.

4. Enzyme agent for property modifying protein material

A fourth aspect of the present invention relates to an enzyme agent for modifying a protein material. As described in the method for producing a property modified protein material according to the second aspect, a peptidylprolyl isomerase alone or a combination of a peptidylprolyl isomerase and a protein modification enzyme can be used for property modifying a protein material. Therefore, the present invention also provides an enzyme agent for property modifying a protein material containing a peptidylprolyl isomerase. The enzyme agent for property modification of the present invention can further contain a protein modification enzyme.

In the enzyme agent for property modifying a protein material of the present invention, the content of the enzyme as an active ingredient, and the types and properties of other components that may be further contained are the same as those of the enzyme agent for protein modification according to the third aspect.

Details of the PPIase, the protein modification enzyme, the property modification mode, the protein material to be a target, and the use method used in the enzyme agent for property modifying a protein material of the present invention are as described in the method for producing a property modified protein material according to the second aspect.

EXAMPLES

Hereinafter, the present invention will be described more specifically with reference to examples, but the present invention is not limited thereto.

<Method for measuring laccase activity>

With respect to the activity of laccase, when 20 $\mu$L of an enzyme solution was added to 20 $\mu$L of a 50 mM solution of 2,2'-Azino-di-[3-ethylbenzthiazoline sulfonate] (ABTS) as a substrate and 160 $\mu$L of a 125 mM sodium phosphate buffer solution, the mixture was reacted at 37°C, and the absorbance at 405 nm after 15 minutes and 0 minute was measured, the amount of enzyme catalyzing the oxidation of 1 $\mu$mol ABTS per minute was defined as 1 unit (U).

<Method for measuring PPlase activity>

The activity of PPlase was measured using Protease-coupled assay. Here, 0.2 mM tetrapeptide (Suc-Ala-Ala-Pro-Phe-pNA) was dissolved in a 0.47 M lithium chloride (LiCl) + trifluoroethanol solution to prepare a substrate solution. Here, 0.3 mg/ml chymotrypsin and an appropriate amount of PPlase were added to the substrate solution, mixed, and treated at 30°C for 3 minutes, then the absorbance at 390 nm (Abs 390 (Ch + PPI)) was measured. As a blank, the measurement was carried out in the same manner as described above under the condition that 0.1 $\mu$M PPlase was not added, and the absorbance (Abs 390 (Ch)) was measured. Assuming that the amount of enzyme releasing 1 $\mu$mol of pNA per minute is 1 U, the amount of pNA is calculated from the following formula.

[Mathematical formula 1]

$$U/mL \text{ or } U/g = \{Abs390(Ch + PPI) - Abs390(Ch)\} \div 0.0133 \div 3 \div n$$

0.0133: coefficient
3: treatment time (min)
n: final concentration of enzyme sample in reaction system (mL/L or g/L)

<Test Example 1: Effect when PPlase and protease are used in combination>

(1) Methods

Here, 10 $\mu$L of 1 mg/mL UMAMIZYME G (Aspergillus oryzae-derived protease, manufactured by Amano Enzyme Inc., 150 u/g) and 10 $\mu$L of 0.1 mg/mL FKBP 12 (microorganism-derived PPlase, manufactured by Sigma, 0.15 mg/ml) were added to 100 $\mu$L of the substrate aqueous solution (Azo casein (manufactured by Sigma) was dissolved at 2% (w/v) in 20 mM Na acetate (pH 6.0)), and the mixture was reacted at 37°C for 0, 10 or 30 minutes, then 240 $\mu$L of a 10% (w/v) TCA solution was added to stop the reaction. After centrifugation, 120 $\mu$L of the supernatant was collected. Here, 140 $\mu$L of 1 N NaOH was added to the recovered supernatant, mixed and caused to develop color, and the absorbance at an OD of 440 nm was measured to evaluate the degradability of azocasein. The decomposing property of azocasein was compared based on a sample (protease only) to which purified water was added instead of FKBP 12.

(2) Results

When the protease was combined with PPlase, azocasein was decomposed more than when only the protease was used. Specifically, as a result of evaluation using a relative value when the degree of decomposition in the case of only protease was taken as 100%, when PPlase was combined, 102% decomposition was observed in the reaction for 10 minutes and 105% decomposition was observed in the reaction for 30 minutes. As the reaction time was longer, there was a difference in the degree of decomposition from the case of only protease. PPlase has only an action of isomerizing a protein, and a chaperone function has only been used so far, but it has been confirmed that the combination of a protease promotes the decomposition by the protease.

<Test Example 2: Effect of using PPlase and protein glutaminase in combination>

(1) Methods

Here, 10 $\mu$L of 25 u/mL PG-500 (Protein glutaminase "Amano" 500, manufactured by Amano Enzyme Inc.) and 10 $\mu$L of 0.1 mg/ml Cyclophilin A (Bovine thymus-derived PPlase, C 7696 manufactured by Sigma) or purified water were added to 1 mL of the substrate solution (10% (w/v) soybean protein (FUJIPRO F, manufactured by Fuji Oil Co., Ltd.) aqueous solution or 10% (w/v) wheat gluten aqueous solution), and the mixture was shaken at 37°C for 10, 60, 120 or 960 minutes, then, 1 mL of a 10% (w/v) TCA solution was added to stop the reaction. The degree of deamidation was evaluated by measuring the amount of ammonia produced in each sample after stopping the reaction using ammonia test Wako (manufactured by FUJIFILM Corporation). Specifically, each post-reaction stop sample was diluted 5-fold with the deproteinized liquid attached to the kit, and then centrifuged to recover the supernatant (removal of protein components). Ammonia nitrogen in the supernatant was developed, and absorbance at 630 nm was compared.

(2) Results

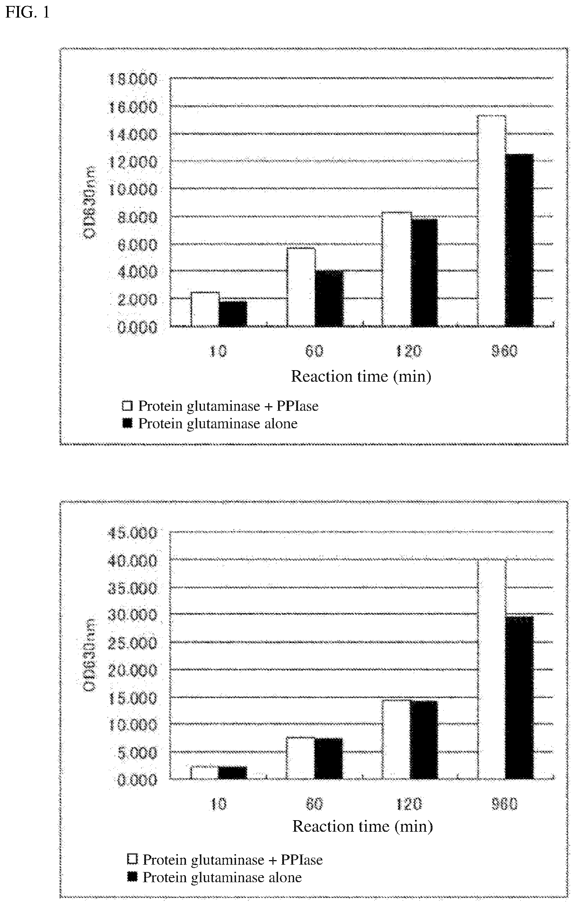

The results are shown in Fig. 1. As shown in Fig. 1, it was confirmed that the deamidation reaction of both soybean protein and wheat protein was promoted when PPIase was combined with protein glutaminase as compared with the case of using only protein glutaminase.

<Test Example 3: Effect when PPIase and pepsin are used in combination>

(1) Methods

A 33 mer peptide (manufactured by PEPTIDE INSTITUTE, INC.) known as a toxic peptide derived from wheat gluten was adjusted to 1.0 mg/ml in water to prepare a substrate solution. To 10 $\mu$l of this substrate solution, 0.5 $\mu$l of a 1 M sodium acetate buffer (pH 4.5), 2.5 $\mu$l of a 5 mg/mL pepsin (manufactured by Sigma) solution, and 10 $\mu$l of a 0.1 mg/mL Cyclophilin A (manufactured by Sigma) solution or 10 $\mu$l of distilled water were added, and the mixture was reacted at 37°C for 1 hour. The residual ratio of the 33 mer peptide in the reaction solution was calculated by ELISA assay using an anti-33 mer peptide antibody. The decomposition property of the peptide was evaluated by a relative amount with the residual ratio of the peptide taken as 100% when only pepsin was used as the enzyme (when Cyclophilin A was not used in combination).

(2) Results

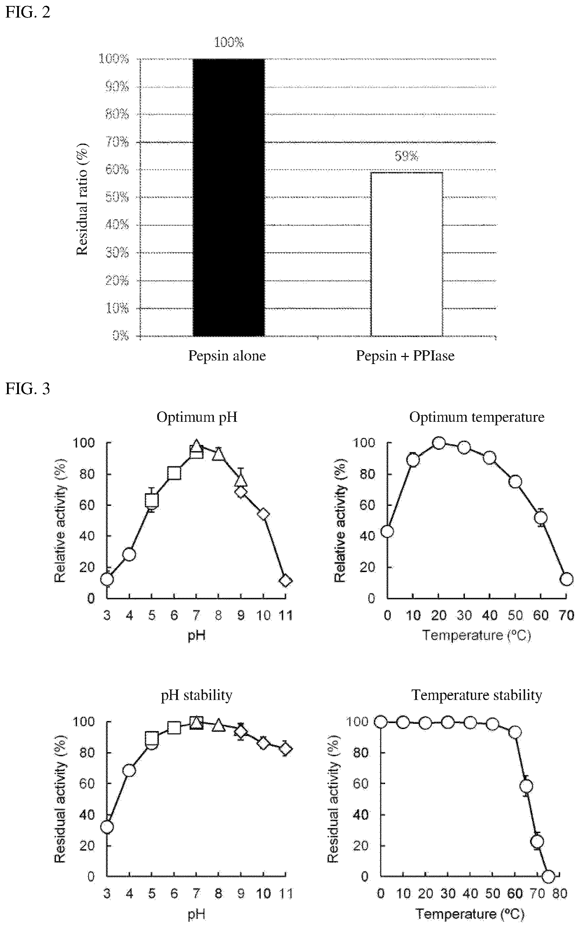

The results are shown in Fig. 2. As shown in Fig. 2, it was confirmed that when PPIase was combined with pepsin, the decomposition of the 33 mer peptide derived from wheat gluten was promoted as compared with the case of only pepsin.

<Test Example 3: Effect when PPIase derived from Streptomyces griseus (SgPPI) and protein glutaminase are used in combination>

(1) Acquisition of PPIase derived from Streptomyces griseus and confirmation of properties

As a result of searching for PPIase having an extracellular secretion signal by BLAST search, it was found that PPIase having an extracellular secretion signal is encoded in the Streptomyces griseus (BSL1) genome. The amino acid sequence of PPIase derived from Streptomyces griseus (hereinafter, it is also described as "SgPPI") is as shown in SEQ ID NO: 1.
SgPPI was expressed in E. coli BL 21 and recovered, and the properties were examined. The optimum pH, optimum temperature, pH stability, and temperature stability of SgPPI are shown in Fig. 3. Although not shown, it was also confirmed that SgPPI had a known PPIase action such as an effect of improving thermal stability or the like.

(2) Methods

For each of a 5% (w/v) wheat gluten aqueous solution, a 5% (w/v) soybean protein aqueous solution, a 5% (w/v) pea protein aqueous solution, a 5% (w/v) milk casein aqueous solution, and a 5% (w/v) egg albumin aqueous solution, 0.1 U of PG-500 (protein glutaminase "Amano" 500, manufactured by Amano Enzyme Inc.) per 1 g of protein, or 0.1 U of PG-500 per 1 g of protein and 174 U of SgPPI per 1 g of protein were added, and the mixture was shaken and reacted at 40°C for 4 hours, then, the reaction was stopped by adding 1 mL of a 10% (w/v) TCA solution. The degree of deamidation was evaluated in the same manner as in Test Example 2.

(3) Results

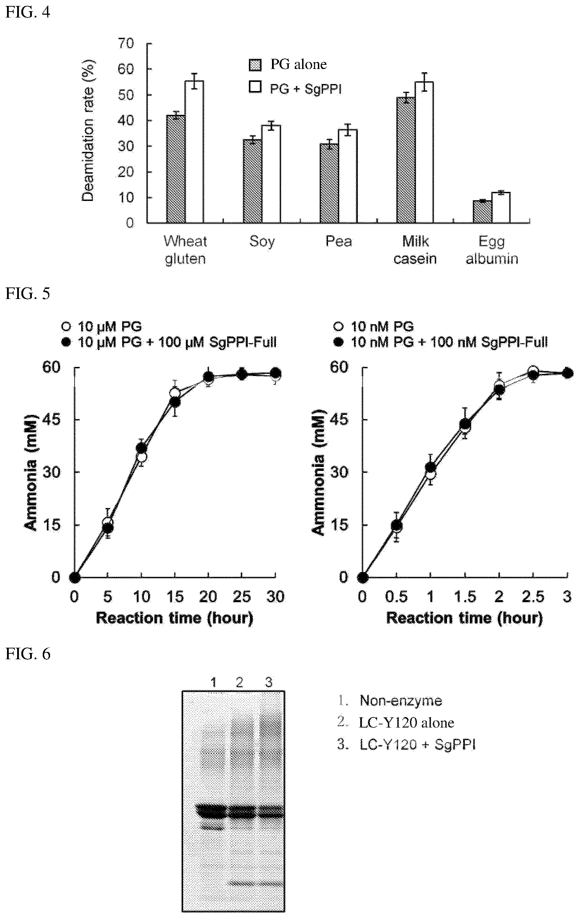

The results are shown in Fig. 4. As shown in Fig. 4, for any of wheat gluten, soybean protein, pea protein, milk casein, and egg albumin, it could be confirmed that the deamidation reaction was promoted when SgPPI was combined with protein glutaminase as compared with the case of using only protein glutaminase.

(4) Supplement: Confirmation of effect on protein glutaminase (PG) when SgPPI is used in combination with PG

A coexistence system of Cbz-Gln-Gly, which is a substrate of protein glutaminase (PG), and PG and a coexistence system of Cbz-Gln-Gly, PG, and SgPPI were constructed, and for each system, PG activity (activity of generating ammonia using Cbz-Gln-Gly as a substrate) was examined over time. The results are shown in Fig. 5. As shown in Figure 5, SgPPI did not improve PG activity against Cbz-Gln-Gly. Therefore, it can be inferred that the effect shown in Fig. 4 is an effect obtained by SgPPI acting on a substrate protein rather than on PG.

<Test Example 4: Effect when SgPPI and laccase are used in combination>

(1) Methods

Here, 2400 U of LC-Y 120 (laccase, manufactured by Amano Enzyme Inc.) per g of protein or 2400 U of LC-Y 120 per g of protein and 174 U of SgPPI per g of protein were added to a 5% (w/v) milk casein aqueous solution, and the mixture was shaken at 40°C for 4 hours, and then the reaction solution was subjected to SDS-PAGE. The crosslinking of proteins and the degree thereof can be confirmed by the presence of a band closer to the origin and the density of the band in SDS-PAGE.

(2) Results

The results are shown in Fig. 6. As shown in Fig. 6, it was found that the amount of crosslinked protein was increased when laccase and SgPPI were used in combination, as compared with the amount of the crosslinked protein obtained when only laccase was used.

<Test Example 5: Effect of using PPIase alone>

(1) Methods

Indian "chickpea" (KOBE GROCERS) protein was suspended at 10 w/v% in ionic water to prepare chickpea milk. SgPPI was added to 10 mL of the obtained chickpea milk so as to be 87 U per 1 g of protein, and the mixture was incubated at 40°C for 24 hours (standing). Thereafter, the properties of the chickpea milk were photographed.

(2) Results

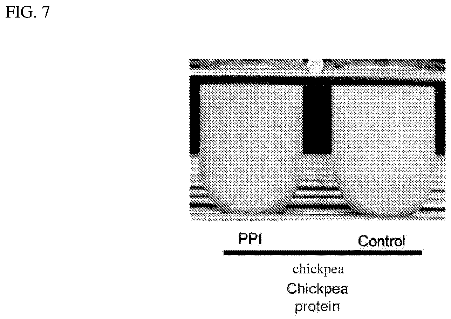

The results are shown in Fig. 7. As shown in Fig. 7, when PPIase was not used (Control), chickpea milk was not dispersed and a large amount of precipitate was generated, but when PPIase was used, chickpea milk was uniformly dispersed, and a property modification effect of improving dispersibility was confirmed.

INDUSTRIAL APPLICABILITY

According to the present invention, a new means capable of property modifying a protein material is provided. Specifically, a protein modification reaction can be regulated (typically, promoted) by allowing a peptidylprolyl isomerase and a protein modification enzyme to act on a protein, and thus the protein material can be modified in property by processing the protein material by combining these enzymes. The protein material can also be modified in property by treating the protein material with a peptidylprolyl isomerase. Such property modified protein materials can be used as industrial materials such as food materials, pharmaceutical materials, industrial materials, and the like, and contribute to the production of industrial products such as food, medicine, industrial products, and the like.

The present invention is not limited to the description of the embodiments and examples of the invention. Various modifications that can be easily conceived by those skilled in the art without departing from the scope of the claims are also included in the present invention. The entire contents of the articles, published patent publications, patent publications, and the like specified in this specification are incorporated herein by reference.

**Claims**

1.  A method for producing a modified protein, comprising a step of allowing a peptidylprolyl isomerase and a protein modification enzyme to act on a protein.

2.  The production method according to claim 1, wherein the treatment of allowing the protein modification enzyme to act and the treatment of allowing the peptidylprolyl isomerase to act are performed simultaneously.

3.  The production method according to claim 1 or 2, wherein the protein modification enzyme is selected from the group consisting of a proteolytic enzyme, a protein side-chain modifying enzyme, and a protein crosslinking enzyme.

4.  The production method according to claim 1 or 2, wherein the protein modification enzyme is selected from the group consisting of a protease, a protein deamidase, a transglutaminase, and an oxidoreductase that catalyzes crosslinking of proteins.

5.  The production method according to any one of claims 1 to 4, wherein the protein is selected from the group consisting of plant proteins derived from beans, grains, nuts, and seeds; milk proteins, egg proteins, blood proteins, muscle proteins and tendon proteins; and proteins derived from microorganisms, algae, and insects.

6.  A method for producing a property modified protein material, comprising a step of treating a food material, a pharmaceutical material, or an industrial material containing a protein with a peptidylprolyl isomerase and a protein modification enzyme.

7.  The production method according to claim 6, wherein the treatment with the protein modification enzyme is performed simultaneously with the treatment with the peptidylprolyl isomerase.

8.  An enzyme agent for protein modification, comprising a peptidylprolyl isomerase and a protein modification enzyme.

9.  The enzyme agent for protein modification according to claim 8, wherein the protein modification enzyme is selected from the group consisting of a proteolytic enzyme, a protein side-chain modifying enzyme, and a protein crosslinking enzyme.

10. The enzyme agent for protein modification according to claim 8, wherein the protein modification enzyme is selected from the group consisting of a protease, a peptidase, a protein deamidase, a transglutaminase, and an oxidoreductase that catalyzes crosslinking of proteins.

11. A method for producing a property modified protein material, comprising a step of treating a food material, a pharmaceutical material, or an industrial material containing a protein with a peptidylprolyl isomerase.

12. An enzyme agent for property modifying a protein material, comprising a peptidylprolyl isomerase.

FIG. 1

FIG. 2

FIG. 3

FIG. 4

FIG. 5

FIG. 6

1. Non-enzyme
2. LC-Y120 alone
3. LC-Y120 + SgPPI

FIG. 7

PPI      Control

chickpea

Chickpea
protein

## INTERNATIONAL SEARCH REPORT

| International application No. |
|---|
| **PCT/JP2021/031039** |

| A. | CLASSIFICATION OF SUBJECT MATTER |
|---|---|

*C12P 21/00*(2006.01)i; *C12N 9/50*(2006.01)i; *C12N 9/78*(2006.01)i; *C12N 9/90*(2006.01)i; *C12P 21/06*(2006.01)i
FI: C12P21/00 Z ZNA; C12P21/06; C12N9/90; C12N9/50; C12N9/78

According to International Patent Classification (IPC) or to both national classification and IPC

| B. | FIELDS SEARCHED |
|---|---|

Minimum documentation searched (classification system followed by classification symbols)

C12P21/00; C12N9/50; C12N9/78; C12N9/90; C12P21/06

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2001-510848 A (MEDICAL RESEARCH COUNCIL) 07 August 2001 (2001-08-07) entirety. in particular, claims, examples | 1-12 |
| X | JP 2001-66309 A (SEKISUI CHEM CO LTD) 16 March 2001 (2001-03-16) entirety. in particular, claims, examples | 11, 12 |
| X | JP 2019-508366 A (F. HOFFMANN-LA ROCHE AG) 28 March 2019 (2019-03-28) entirety. in particular, claims, examples | 1-12 |
| Y | WO 2006/082922 A1 (SAN-EI GEN F.F.I., INC) 10 August 2006 (2006-08-10) claims, examples | 1-12 |
| Y | JP 2018-74967 A (NISSHIN PHARMA INC) 17 May 2018 (2018-05-17) claims, examples | 1-12 |
| Y | WO 2019/083795 A1 (PFENEX INC.) 02 May 2019 (2019-05-02) paragraphs [0102], [0103] | 1-12 |

| ☐ Further documents are listed in the continuation of Box C. | ☑ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **08 November 2021** | **16 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
|---|
| **PCT/JP2021/031039** |

| **Box No. I** | **Nucleotide and/or amino acid sequence(s) (Continuation of item 1.c of the first sheet)** |
|---|---|

1.  With regard to any nucleotide and/or amino acid sequence disclosed in the international application, the international search was carried out on the basis of a sequence listing:

    a.  ☑  forming part of the international application as filed:

          ☑   in the form of an Annex C/ST.25 text file.

          ☐   on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐   in the form of an Annex C/ST.25 text file (Rule 13*ter*.1(a)).

          ☐   on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

INTERNATIONAL SEARCH REPORT
Information on patent family members

International application No.

**PCT/JP2021/031039**

| Patent document cited in search report | | | Publication date (day/month/year) | Patent family member(s) | | | Publication date (day/month/year) |
|---|---|---|---|---|---|---|---|
| JP | 2001-510848 | A | 07 August 2001 | US | 2003/0077692 | A1 | |
| | | | | claims, examples | | | |
| | | | | WO | 1999/005163 | A1 | |
| | | | | EP | 998485 | A1 | |
| JP | 2001-66309 | A | 16 March 2001 | (Family: none) | | | |
| JP | 2019-508366 | A | 28 March 2019 | US | 2019/0381181 | A1 | |
| | | | | claims, examples | | | |
| | | | | WO | 2017/102759 | A1 | |
| | | | | EP | 3390424 | A1 | |
| | | | | KR | 10-2018-0087431 | A | |
| | | | | CN | 108699104 | A | |
| WO | 2006/082922 | A1 | 10 August 2006 | JP | 4637896 | B2 | |
| JP | 2018-74967 | A | 17 May 2018 | (Family: none) | | | |
| WO | 2019/083795 | A1 | 02 May 2019 | US | 2019/0127744 | A1 | |
| | | | | EP | 3700915 | A1 | |
| | | | | KR | 10-2020-0073280 | A | |
| | | | | CN | 111372941 | A | |
| | | | | JP | 2021-501157 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2002525120 PCT
- JP 2004215591 A
- JP 2005046066 A
- JP H2124100 A
- JP H11075837 A
- WO 2015133590 A

### Non-patent literature cited in the description

- *Biochim Biophys Acta,* October 2015, vol. 1850 (10), 2017-2034
- *Biomed Biochim Acta,* 1984, vol. 43 (10), 1101-11
- *Biochim Biophys Acta,* October 2015, vol. 1850 (10), 2017-2034
- *Protein Engineering, Design and Selection,* February 2008, vol. 21 (2), 83-89
- **TATIANA A. TATSUSOVA ; THOMAS L. MADDEN.** *FEMS Microbiol. Lett.,* 1999, vol. 174, 247-250
- **S YAMAGUCHI 1 ; D J JEENES ; D B ARCHER.** Protein-glutaminase From Chryseobacterium Proteolyticum, an Enzyme That Deamidates Glutaminyl Residues in Proteins.Purification, Characterization and Gene Cloning. *Eur J Biochem,* 2001, vol. 268 (5), 1410
- **RUIDAN QU ; XIAOYU ZHU ; MIN TIAN ; YINGJIE LIU ; WENJUAN YAN ; JIAN YE ; HONGLIANG GAO ; JING HUANG YANG.** Complete Genome Sequence and Characterization of a Protein-Glutaminase Producing Strain, Chryseobacterium proteolyticum QSH 1265. *Front Microbiol,* 2018, vol. 9, 1975
- Molecular Approaches to Improving Food Quality and Safety. Van Nostrand Reinhold, 1992, 37